# EUROPEAN PATENT SPECIFICATION

(11) **EP 2 984 598 B1**
(45) Date of publication and mention of the grant of the patent: **04.12.2024**
(21) Application number: 14716060.0
(22) Date of filing: 05.03.2014
(51) Int. Cl.: G16B 20/10

(54) **SYSTEMS AND METHODS FOR DETERMINING COPY NUMBER VARIATION**
SYSTEME UND VERFAHREN ZUR BESTIMMUNG VON KOPIENZAHLVARIANTEN
SYSTÈMES ET PROCÉDÉS DESTINÉS À DÉFINIR UNE VARIATION DU NOMBRE DE COPIE

(30) Priority: 06.03.2013 US 201361773584 P
(43) Date of publication of application: 17.02.2016
(60) Divisional application: 24210117.8
(73) Proprietor: Life Technologies Corporation, Carlsbad, CA 92008 (US)
(72) Inventor: KONVICKA, Karel, San Francisco, CA 94114 (US); VEITCH, James, Carlsbad, CA 92008 (US)
(74) Representative: Hoffmann Eitle
(86) International application number: PCT/US2014/020516
(87) International publication number: WO 2014/138153

(56) References cited:
- US-A1- 2011 177 517
- US-A1- 2012 046 877
- US-A1- 2012 095 697
- US-A1- 2012 184 449
- SEQUENCING TARGETED: "Technical Note Multiplexing Targeted Sequencing using Barcodes Experimental Design", 16 January 2013 (2013-01-16), XP055978445, Retrieved from the Internet <URL:https://www.pacb.com/wp-content/uploads/TN_Multiplexing_Targeted_Sequencing_Using_Barcodes.pdf> [retrieved on 20221107]

## Description

### BACKGROUND

### FIELD

The present disclosure generally relates to the field of nucleic acid sequencing including systems and methods for determining copy number variation.

### RELATED APPLICATIONS

This application is related to U.S. Provisional Application No. 61/773,584 filed March 6, 2013.

### INTRODUCTION

Upon completion of the Human Genome Project, one focus of the sequencing industry has shifted to finding higher throughput and/or lower cost nucleic acid sequencing technologies, sometimes referred to as "next generation" sequencing (NGS) technologies. In making sequencing higher throughput and/or less expensive, the goal is to make the technology more accessible. These goals can be reached through the use of sequencing platforms and methods that provide sample preparation for samples of significant complexity, sequencing larger numbers of samples in parallel (for example through use of barcodes and multiplex analysis) , and/or processing high volumes of information efficiently and completing the analysis in a timely manner. Various methods, such as, for example, sequencing by synthesis, sequencing by hybridization, and sequencing by ligation are evolving to meet these challenges.

Ultra-high throughput nucleic acid sequencing systems incorporating NGS technologies typically produce a large number of short sequence reads. Sequence processing methods should desirably assemble and/or map a large number of reads quickly and efficiently, such as to minimize use of computational resources. For example, data arising from sequencing of a mammalian genome can result in tens or hundreds of millions of reads that typically need to be assembled before they can be further analyzed to determine their biological, diagnostic and/or therapeutic relevance.

Exemplary applications of NGS technologies include, but are not limited to: genomic variant detection, such as insertions/deletions, copy number variations, single nucleotide polymorphisms, etc., genomic resequencing, gene expression analysis and genomic profiling.

Copy number variations (CNVs) can be indicative of large scale chromosomal rearrangements, such as large insertions or deletions, which can be common found in cancer tissue. In some cases, entire chromosomes can be lost or duplicated (aneuploidy), which is a common cause of genetic disorders, such as Down syndrome (trisomy 21), cat eye syndrome (trisomy 22), Williams syndrome (monosomy 7), and various others. Identifying copy number variations can help understand and diagnose cancer and aneuploid genetic disorders.
United States Patent Application Publication US 2012/0095697 discloses a method for determining a copy number of a genomic region at a detection position of a target sequence in a sample. Genomic regions of the target sequence are sequenced and a sequence coverage is obtained at the detection position from which the copy number is calculated.
United States Patent Application Publication US 2012/0046877 discloses a method for analyzing a copy number variation in chromosomal regions of a sample. A target sequence is sequenced and a read coverage is determined for non-overlapping window regions from which the copy number variation is calculated.

From the foregoing it will be appreciated that a need exists for systems and methods that can determine copy number variations.

### DRAWINGS

For a more complete understanding of the principles disclosed herein, and the advantages thereof, reference is now made to the following descriptions taken in conjunction with the accompanying drawings, in which:
Figure 1 is a block diagram that illustrates an exemplary computer system, in accordance with various embodiments.
Figure 2 is a schematic diagram of an exemplary system for reconstructing a nucleic acid sequence, in accordance with various embodiments.
Figure 3 is a schematic diagram of an exemplary genetic analysis system, in accordance with various embodiments.
Figure 4 is a flow diagram illustrating an exemplary method of identifying copy number variations, in accordance with various embodiments.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

### SUMMARY OF THE INVENTION

The invention is defined in the independent claims.

### SUMMARY OF THE DISCLOSURE

The following "various embodiments" described herein relate to examples of systems and methods for detecting low frequency variants.

In various embodiments, a method of identifying copy number variations in reads includes mapping reads to a reference genome, computing coverage for a plurality of tiles, and normalizing the coverage for a tile based on a coverage mode across the plurality of tiles. The method further includes determining a score for the plurality of tiles being in a plurality of ploidy states, determining a maximum score path across the tiles and through the ploidy states, and providing a copy number determination based on the maximum likelihood path.

In various embodiments, the coverage mode can be corrected for GC bias. In various embodiments, the score for a tile being in a ploidy state is based on the difference between the normalized coverage and a scaled baseline coverage adjusted to the explored ploidy state.

In various embodiments, the score is a likelihood function. The likelihood can be determined using the equation L = N( S-C, 0, Sd ), where S is the normalized sample coverage for the tile, C is a scaled baseline coverage for the tile, and Sd is the standard deviation of the coverage difference.

In various embodiments, the maximum score path can be determined using dynamic programming algorithm. In various embodiments, the method can further include determining a score ratio of the maximum score path to the expected ploidy state. In various embodiments, the method can further include determining a score ratio of the maximum score path to the most likely neighboring state.

In various embodiments, a system for identifying duplicate reads includes a mapping engine, and a copy number analysis module. The mapping engine is operable to map reads to a reference genome to determine a genomic start position and an flow end position. The copy number analysis module includes a processing engine and
a copy number variant caller. The processing engine operable to determine coverages for tiles and normalize the coverages based on a coverage mode and GC content bias. The copy number variant caller operable to determine a score for tiles to be present in a plurality of ploidy states, and determine a maximum score path across the tiles through the ploidy states.

In various embodiments, the score is a likelihood function. The likelihood for a tile being in a ploidy state can be based on the difference between the normalized coverage and a scaled baseline coverage scaled to the ploidy state. The likelihood can be determined using the equation L = N( S-C, 0, Sd ), where S is the normalized sample coverage for the tile, C is the scaled baseline coverage for the tile, and Sd is the standard deviation of the coverage difference.

In various embodiments, the maximum score path is determined using dynamic programming algorithm. In various embodiments, the copy number analysis module can further include a post-processing module operable to determine a score ratio of the maximum score path to the expected ploidy state. In various embodiments, the copy number analysis module can further include a post-processing module operable to determine a score ratio of the maximum score path to the most likely neighboring ploidy state.

In various embodiments, a method of identifying copy number variations in reads includes performing a multiplex amplification on a sample to generate a set of sample amplicons, and performing a multiplex amplification on a matched control to generate a set of control amplicons. The method further includes joining adaptors having a first barcode sequence to the sample amplicons to create a sample library, joining adaptors having a second barcode sequence to the control amplicons to create a control library, and sequencing the sample and control libraries substantially simultaneously to avoid intra-run sequencing variations to generate a plurality of reads. Additionally, the method includes identifying reads as either sample reads or control reads based on the presence of the first or second barcode sequence, and mapping the sample reads and control reads to a reference genome. Further, the method includes computing a sample coverage for a plurality of tiles based on the sample reads that map to the tiles, computing a baseline coverage for the tiles based on the control reads that map to the tiles, and normalizing the sample coverage and baseline coverage for a tile based on a sample coverage mode or a control coverage mode across the plurality of tiles. In various embodiments, the sample coverage mode and the control coverage mode can be corrected for GC bias. The method further includes determining a score for the plurality of tiles being in a plurality of ploidy states based on the normalized sample coverage and the baseline coverage for the tiles, determining a maximum likelihood path across the tiles and through the ploidy states; and providing a copy number determination based on the maximum likelihood path.

In various embodiments, the score for a tile being in a ploidy state is based on the difference between the normalized coverage and a scaled baseline coverage adjusted to the explored ploidy state. The score is a likelihood function. The likelihood is determined using the equation L = N( S-C, 0, Sd ), where S is the normalized sample coverage for the tile, C is a scaled baseline coverage for the tile, and Sd is the standard deviation of the coverage difference.

In various embodiments, the method can further include determining a score ratio of the maximum score path to the expected ploidy state. In various embodiments, the method can further include determining a score ratio of the maximum score path to the most likely neighboring state.

The section headings used herein are for organizational purposes only and are not to be construed as limiting the described subject matter in any way.

In this detailed description of the various embodiments, for purposes of explanation, numerous specific details are set forth to provide a thorough understanding of the embodiments disclosed. One skilled in the art will appreciate, however, that these various embodiments may be practiced with or without these specific details. In other instances, structures and devices are shown in block diagram form. described otherwise, all technical and scientific terms used herein have a meaning as is commonly understood by one of ordinary skill in the art to which the various embodiments described herein belongs.

It will be appreciated that there is an implied "about" prior to the temperatures, concentrations, times, number of bases, coverage, etc. discussed in the present teachings, such that slight and insubstantial deviations are within the scope of the present teachings. In this application, the use of the singular includes the plural unless specifically stated otherwise. Also, the use of "comprise", "comprises", "comprising", "contain", "contains", "containing", "include", "includes", and "including" are not intended to be limiting. It is to be understood that both the foregoing general description and the following detailed description are exemplary and explanatory only and are not restrictive of the present teachings.

As used herein, "a" or "an" also may refer to "at least one" or "one or more." Also, the use of "or" is inclusive, such that the phrase "A or B" is true when "A" is true, "B" is true, or both "A" and "B" are true.

Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. The techniques and procedures described herein are generally performed according to conventional methods well known in the art and as described in various general and more specific references that are cited and discussed throughout the instant specification. *See, e.g.,* Sambrook et al., Molecular Cloning: A Laboratory Manual (Third ed., Cold Spring Harbor Laboratory Press, Cold Spring Harbor, N.Y. 2000). The nomenclatures utilized in connection with, and the laboratory procedures and techniques described herein are those well known and commonly used in the art.

A "system" sets forth a set of components, real or abstract, comprising a whole where each component interacts with or is related to at least one other component within the whole.

A "biomolecule" may refer to any molecule that is produced by a biological organism, including large polymeric molecules such as proteins, polysaccharides, lipids, and nucleic acids (DNA and RNA) as well as small molecules such as primary metabolites, secondary metabolites, and other natural products.

The phrase "next generation sequencing" or NGS refers to sequencing technologies having increased throughput as compared to traditional Sanger- and capillary electrophoresis-based approaches, for example with the ability to generate hundreds of thousands of relatively small sequence reads at a time. Some examples of next generation sequencing techniques include, but are not limited to, sequencing by synthesis, sequencing by ligation, and sequencing by hybridization. More specifically, the Personal Genome Machine (PGM) of Life Technologies Corp. provides massively parallel sequencing with enhanced accuracy. The PGM System and associated workflows, protocols, chemistries, etc. are described in more detail in U.S. Patent Application Publication No. 2009/0127589 and No. 2009/0026082.

The phrase "sequencing run" refers to any step or portion of a sequencing experiment performed to determine some information relating to at least one biomolecule (e.g., nucleic acid molecule).

The phase "base space" refers to a representation of the sequence of nucleotides. The phase "flow space" refers to a representation of the incorporation event or non-incorporation event for a particular nucleotide flow. For example, flow space can be a series of values representing a nucleotide incorporation events (such as a one, "1") or a non-incorporation event (such as a zero, "0") for that particular nucleotide flow. Nucleotide flows having a non-incorporation event can be referred to as empty flows, and nucleotide flows having a nucleotide incorporation event can be referred to as positive flows. It should be understood that zeros and ones are convenient representations of a non-incorporation event and a nucleotide incorporation event; however, any other symbol or designation could be used alternatively to represent and/or identify these events and non-events. In particular, when multiple nucleotides are incorporated at a given position, such as for a homopolymer stretch, the value can be proportional to the number of nucleotide incorporation events and thus the length of the homopolymer stretch.

DNA (deoxyribonucleic acid) is a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. Certain pairs of nucleotides specifically bind to one another in a complementary fashion (called complementary base pairing). That is, adenine (A) pairs with thymine (T) (in the case of RNA, however, adenine (A) pairs with uracil (U)), and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "nucleic acid sequence," "genomic sequence," "genetic sequence," or "fragment sequence," or "nucleic acid sequencing read" denotes any information or data that is indicative of the order of the nucleotide bases (e.g., adenine, guanine, cytosine, and thymine/uracil) in a molecule (e.g., whole genome, whole transcriptome, exome, oligonucleotide, polynucleotide, fragment, etc.) of DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

A "polynucleotide", "nucleic acid", or "oligonucleotide" refers to a linear polymer of nucleosides (including deoxyribonucleosides, ribonucleosides, or analogs thereof) joined by intemucleosidic linkages. Typically, a polynucleotide comprises at least three nucleosides. Usually oligonucleotides range in size from a few monomeric units, e.g. 3-4, to several hundreds of monomeric units. Whenever a polynucleotide such as an oligonucleotide is represented by a sequence of letters, such as "ATGCCTG," it will be understood that the nucleotides are in 5'->3' order from left to right and that "A" denotes deoxyadenosine, "C" denotes deoxycytidine, "G" denotes deoxyguanosine, and "T" denotes thymidine, unless otherwise noted. The letters A, C, G, and T may be used to refer to the bases themselves, to nucleosides, or to nucleotides comprising the bases, as is standard in the art.

As used herein, a "somatic variation" or "somatic mutation" can refer to a variation in genetic sequence that results from a mutation that occurs in a non-germline cell. The variation can be passed on to daughter cells through mitotic division. This can result in a group of cells having a genetic difference from the rest of the cells of an organism. Additionally, as the variation does not occur in a germline cell, the mutation may not be inherited by progeny organisms.

As defined herein "multiplex amplification" refers to selective and non-random amplification of two or more target sequences within a sample using at least one target-specific primer. In some embodiments, multiplex amplification is performed such that some or all of the target sequences are amplified within a single reaction vessel. The "plexy" or "plex" of a given multiplex amplification refers generally to the number of different target-specific sequences that are amplified during that single multiplex amplification. In some embodiments, the plexy can be about 12-plex, 24-plex, 48-plex, 96-plex, 192-plex, 384-plex, 768-plex, 1536-plex, 3072-plex, 6144-plex or higher.

### MULTIPLEX AMPLIFICATION METHODS:

In various embodiments, target nucleic acids generated by the amplification of multiple target-specific sequences from a population of nucleic acid molecules can be sequenced. In some embodiments, the amplification can include hybridizing one or more target-specific primer pairs to the target sequence, extending a first primer of the primer pair, denaturing the extended first primer product from the population of nucleic acid molecules, hybridizing to the extended first primer product the second primer of the primer pair, extending the second primer to form a double stranded product, and digesting the target-specific primer pair away from the double stranded product to generate a plurality of amplified target sequences. In some embodiments, the amplified target sequences can be ligated to one or more adapters. In some embodiments, the adapters can include one or more DNA barcodes or tagging sequences. In some embodiments, the amplified target sequences once ligated to an adapter can undergo a nick translation reaction and/or further amplification to generate a library of adapter-ligated amplified target sequences. Exemplary methods of multiplex amplification are described in U.S. Application No. 13/458,739 filed November 12, 2012 and titled "Methods and Compositions for Multiplex PCR",

In various embodiments, the method of performing multiplex PCR amplification includes contacting a plurality of target-specific primer pairs having a forward and reverse primer, with a population of target sequences to form a plurality of template/primer duplexes; adding a DNA polymerase and a mixture of dNTPs to the plurality of template/primer duplexes for sufficient time and at sufficient temperature to extend either (or both) the forward or reverse primer in each target-specific primer pair via template-dependent synthesis thereby generating a plurality of extended primer product/template duplexes; denaturing the extended primer product/template duplexes; annealing to the extended primer product the complementary primer from the target-specific primer pair; and extending the annealed primer in the presence of a DNA polymerase and dNTPs to form a plurality of target-specific double-stranded nucleic acid molecules.

### ADAPTOR-JOINING METHODS:

In some embodiments, the present teachings are directed to methods for preparing a library of polynucleotide constructs which can include an adaptor-joining step. In some embodiments, a plurality of polynucleotide fragments can include at least two polynucleotide fragments that are joined to one or more nucleic acid adaptors by hybridization (e.g., with or without a primer extension reaction) or enzymatic ligation (e.g., a ligase reaction) to generate adaptor-fragment constructs. In some embodiments, one end or both ends of polynucleotide fragments can be joined to at least one type of adaptor. One or both ends of a polynucleotide fragment can be joined to at least one nucleic acid adaptor, including barcoded adaptors, sequencing primer adaptors, amplification primer adaptors, universal adaptors, blocking oligonucleotide adaptors and/or others.

In some embodiments, an adaptor can include nucleotide sequences that are complementary to sequencing primers (e.g., P1, P2 and/or A), amplification primers, universal sequences and/or barcode sequences. For example, released mate pair constructs can be joined at each end to a different sequencing adaptor to prepare a nucleic acid library for sequencing with SOLiD^{™} sequencing reactions (WO 2006/084131) or sequencing with ion-sensitive sequencing reactions (e.g., Ion Torrent PGM^{™} and Proton^{™} sequencers from Life Technologies Corporation, see for example U.S. Patent Publication Nos. 2010/0301398, 2010/0300895, 2010/0300559, 2010/0197507, 2010/0137143, 2009/0127589; and 2009/0026082 .

### BARCODED ADAPTOR SEQUENCES

In some embodiments, the present teachings are directed to methods for preparing a library of polynucleotide constructs which can include joining at least one end of a plurality of polynucleotide fragments to an adaptor having a barcode sequence. A barcode sequence can be a selected sequence of nucleotide bases (e.g. adenine, guanine, cytosine, thymine, uracil, inosine, or analogs thereof) in the polynucleotide strand that serves to identify the polynucleotide strand and/or distinguish it from other polynucleotide strands (e.g. those containing a different target sequence of interest). In some embodiments, a barcode adaptor can include a unique identification sequence (e.g., barcode sequence). A barcode sequence can be used for various purposes, such as tracking, sorting, and/or identifying the samples.

Because different barcode sequences can be associated with different polynucleotide strands, these barcode sequences may be useful in multiplexed sequencing of different samples. In some embodiments, a barcode adaptor can be used for constructing multiplex nucleic acid libraries. In some embodiments, one or more barcode sequences can allow identification of a particular adaptor among a mixture of different adaptors having different barcodes sequences. For example, a mixture can include 2, 3, 4, 5, 6, 7-10, 10-50, 50-100, 100-200, 200-500, 500-1000, or more different adaptors having unique barcode sequences. Examples of various adaptors having barcode sequences can be found in WO 2012/044847.

In various high throughput DNA sequencing technologies (such as sequencing-by-synthesis) it is desirable to permit sequencing of different samples that are pooled together for simultaneous analysis (sometimes referred to as multiplexed sequencing).

When carrying out multiplexed sequencing, it is generally desirable to identify the origin of each sample, and this may require that the sequencing data be deconvolved for each sample. In particular, it can be desirable to uniquely identify the source of the sequence data derived from a multiplex sample (for example, to identify a particular nucleic acid species associated with different sample populations). One approach to facilitate sample identification is the use of unique nucleic acid identifier sequences (barcode adaptors) that are embedded within the sample construct so that sequencing data can be correctly identified or associated with its source sample.

### COMPUTER-IMPLEMENTED SYSTEM

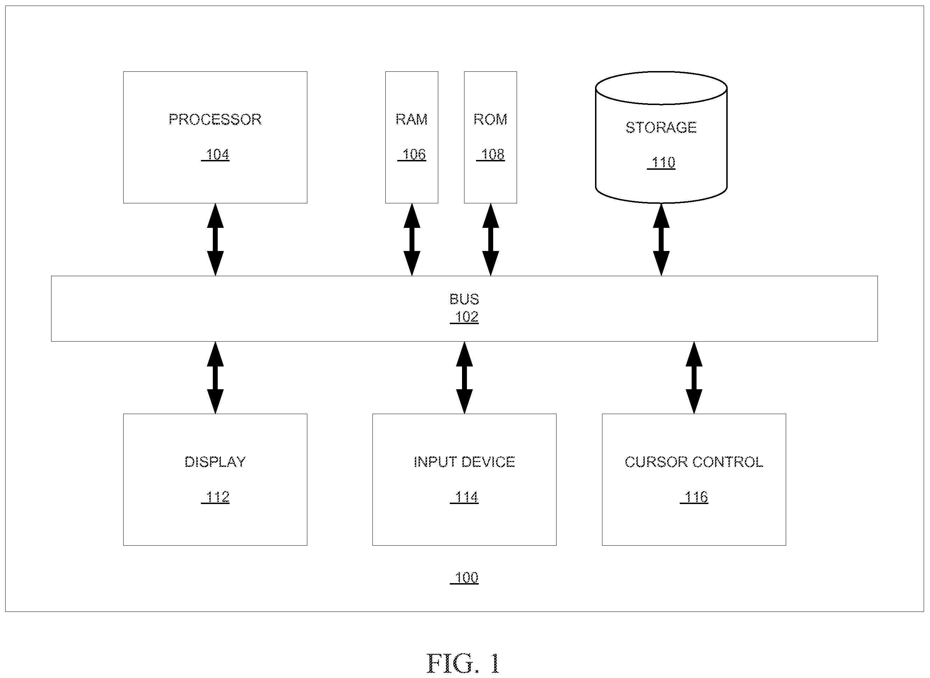

Figure 1 is a block diagram that illustrates a computer system 100, upon which embodiments of the present teachings may be implemented. In various embodiments, computer system 100 can include a bus 102 or other communication mechanism for communicating information, and a processor 104 coupled with bus 102 for processing information. In various embodiments, computer system 100 can also include a memory 106, which can be a random access memory (RAM) or other dynamic storage device, coupled to bus 102 for determining base calls, and instructions to be executed by processor 104. Memory 106 also can be used for storing temporary variables or other intermediate information during execution of instructions to be executed by processor 104. In various embodiments, computer system 100 can further include a read only memory (ROM) 108 or other static storage device coupled to bus 102 for storing static information and instructions for processor 104. A storage device 110, such as a magnetic disk or optical disk, can be provided and coupled to bus 102 for storing information and instructions.

In various embodiments, processor 104 can include a plurality of logic gates. The logic gates can include AND gates, OR gates, NOT gates, NAND gates, NOR gates, EXOR gates, EXNOR gates, or any combination thereof. An AND gate can produce a high output only if all the inputs are high. An OR gate can produce a high output if one or more of the inputs are high. A NOT gate can produce an inverted version of the input as an output, such as outputting a high value when the input is low. A NAND (NOT-AND) gate can produce an inverted AND output, such that the output will be high if any of the inputs are low. A NOR (NOT-OR) gate can produce an inverted OR output, such that the NOR gate output is low if any of the inputs are high. An EXOR (Exclusive-OR) gate can produce a high output if either, but not both, inputs are high. An EXNOR (Exclusive-NOR) gate can produce an inverted EXOR output, such that the output is low if either, but not both, inputs are high.

**Table 1: Logic Gates Truth Table**

| IN PUTS | | OUTPUTS | | | | | | |
|---|---|---|---|---|---|---|---|---|
| A | B | NOT A | AND | NAND | OR | NOR | EXOR | EXNOR |
| 0 | 0 | 1 | 0 | 1 | 0 | 1 | 0 | 1 |
| 0 | 1 | 1 | 0 | 1 | 1 | 0 | 1 | 0 |
| 1 | 0 | 0 | 0 | 1 | 1 | 0 | 1 | 0 |
| 1 | 1 | 0 | 1 | 0 | 1 | 0 | 0 | 1 |

One of skill in the art would appreciate that the logic gates can be used in various combinations to perform comparisons, arithmetic operations, and the like. Further, one of skill in the art would appreciate how to sequence the use of various combinations of logic gates to perform complex processes, such as the processes described herein.

In an example, a 1-bit binary comparison can be performed using a XNOR gate since the result is high only when the two inputs are the same. A comparison of two multi-bit values can be performed by using multiple XNOR gates to compare each pair of bits, and the combining the output of the XNOR gates using and AND gates, such that the result can be true only when each pair of bits have the same value. If any pair of bits does not have the same value, the result of the corresponding XNOR gate can be low, and the output of the AND gate receiving the low input can be low.

In another example, a 1-bit adder can be implemented using a combination of AND gates and XOR gates. Specifically, the 1-bit adder can receive three inputs, the two bits to be added (A and B) and a carry bit (Cin), and two outputs, the sum (S) and a carry out bit (Cout). The Cin bit can be set to 0 for addition of two one bit values, or can be used to couple multiple 1-bit adders together to add two multi-bit values by receiving the Cout from a lower order adder. In an exemplary embodiment, S can be implemented by applying the A and B inputs to a XOR gate, and then applying the result and Cin to another XOR gate. Cout can be implemented by applying the A and B inputs to an AND gate, the result of the A-B XOR from the SUM and the Cin to another AND, and applying the input of the AND gates to a XOR gate.

**Table 2: 1-bit Adder Truth Table**

| INPUTS | | | OUTPUTS | |
|---|---|---|---|---|
| A | B | Cin | S | Cout |
| 0 | 0 | 0 | 0 | 0 |
| 1 | 0 | 0 | 0 | 1 |
| 0 | 1 | 0 | 0 | 1 |
| 1 | 1 | 0 | 1 | 0 |
| 0 | 0 | 1 | 0 | 1 |
| 1 | 0 | 1 | 1 | 0 |
| 0 | 1 | 1 | 1 | 0 |
| 1 | 1 | 1 | 1 | 1 |

In various embodiments, computer system 100 can be coupled via bus 102 to a display 112, such as a cathode ray tube (CRT) or liquid crystal display (LCD), for displaying information to a computer user. An input device 114, including alphanumeric and other keys, can be coupled to bus 102 for communicating information and command selections to processor 104. Another type of user input device is a cursor control 116, such as a mouse, a trackball or cursor direction keys for communicating direction information and command selections to processor 104 and for controlling cursor movement on display 112. This input device typically has two degrees of freedom in two axes, a first axis (i.e., x) and a second axis (i.e., y), that allows the device to specify positions in a plane.

A computer system 100 can perform the present teachings. Consistent with certain implementations of the present teachings, results can be provided by computer system 100 in response to processor 104 executing one or more sequences of one or more instructions contained in memory 106. Such instructions can be read into memory 106 from another computer-readable medium, such as storage device 110. Execution of the sequences of instructions contained in memory 106 can cause processor 104 to perform the processes described herein. In various embodiments, instructions in the memory can sequence the use of various combinations of logic gates available within the processor to perform the processes describe herein. Alternatively hard-wired circuitry can be used in place of or in combination with software instructions to implement the present teachings. In various embodiments, the hard-wired circuitry can include the necessary logic gates, operated in the necessary sequence to perform the processes described herein. Thus implementations of the present teachings are not limited to any specific combination of hardware circuitry and software.

The term "computer-readable medium" as used herein refers to any media that participates in providing instructions to processor 104 for execution. Such a medium can take many forms, including but not limited to, non-volatile media, volatile media, and transmission media. Examples of non-volatile media can include, but are not limited to, optical or magnetic disks, such as storage device 110. Examples of volatile media can include, but are not limited to, dynamic memory, such as memory 106. Examples of transmission media can include, but are not limited to, coaxial cables, copper wire, and fiber optics, including the wires that comprise bus 102.

Common forms of non-transitory computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

In accordance with various embodiments, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

### NUCLEIC ACID SEQUENCING PLATFORMS

Nucleic acid sequence data can be generated using various techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

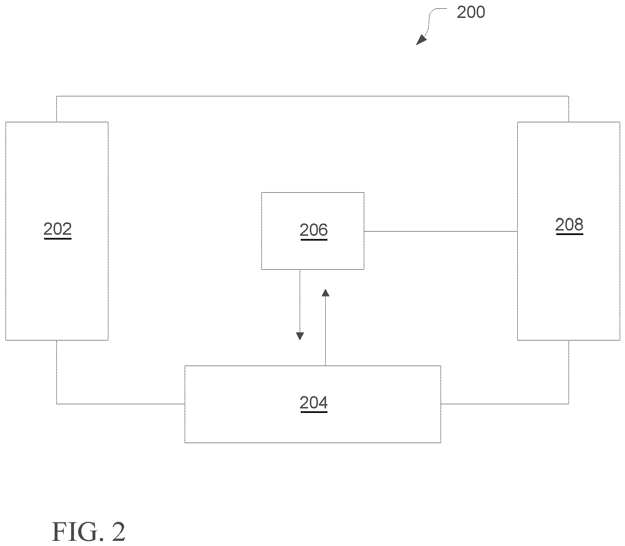

Various embodiments of nucleic acid sequencing platforms, such as a nucleic acid sequencer, can include components as displayed in the block diagram of Figure 2. According to various embodiments, sequencing instrument 200 can include a fluidic delivery and control unit 202, a sample processing unit 204, a signal detection unit 206, and a data acquisition, analysis and control unit 208. Various embodiments of instrumentation, reagents, libraries and methods used for next generation sequencing are described in U.S. Patent Application Publication No. 2009/0127589 and No. 2009/0026082 . Various embodiments of instrument 200 can provide for automated sequencing that can be used to gather sequence information from a plurality of sequences in parallel, such as substantially simultaneously.

In various embodiments, the fluidics delivery and control unit 202 can include reagent delivery system. The reagent delivery system can include a reagent reservoir for the storage of various reagents. The reagents can include RNA-based primers, forward/reverse DNA primers, oligonucleotide mixtures for ligation sequencing, nucleotide mixtures for sequencing-by-synthesis, optional ECC oligonucleotide mixtures, buffers, wash reagents, blocking reagent, stripping reagents, and the like. Additionally, the reagent delivery system can include a pipetting system or a continuous flow system which connects the sample processing unit 204 with the reagent reservoir.

In various embodiments, the sample processing unit 204 can include a sample chamber, such as flow cell, a substrate, a micro-array, a multi-well tray, or the like. The sample processing unit 204 can include multiple lanes, multiple channels, multiple wells, or other means of processing multiple sample sets substantially simultaneously. Additionally, the sample processing unit 204 can include multiple sample chambers to enable processing of multiple runs simultaneously. In particular embodiments, the system can perform signal detection on one sample chamber while substantially simultaneously processing another sample chamber. Additionally, the sample processing unit 204 can include an automation system for moving or manipulating the sample chamber.

In various embodiments, the signal detection unit 206 can include an imaging or detection sensor. For example, the imaging or detection sensor can include a CCD, a CMOS, an ion or chemical sensor, such as an ion sensitive layer overlying a CMOS or FET, a current or voltage detector, or the like. The signal detection unit 206 can include an excitation system to cause a probe, such as a fluorescent dye, to emit a signal. The excitation system can include an illumination source, such as arc lamp, a laser, a light emitting diode (LED), or the like. In particular embodiments, the signal detection unit 206 can include optics for the transmission of light from an illumination source to the sample or from the sample to the imaging or detection sensor.
Alternatively, the signal detection unit 206 may provide for electronic or non-photon based methods for detection and consequently not include an illumination source. In various embodiments, electronic-based signal detection may occur when a detectable signal or species is produced during a sequencing reaction. For example, a signal can be produced by the interaction of a released byproduct or moiety, such as a released ion, such as a hydrogen ion, interacting with an ion or chemical sensitive layer. In other embodiments a detectable signal may arise as a result of an enzymatic cascade such as used in pyrosequencing (see, for example, U.S. Patent Application Publication No. 2009/0325145 ) where pyrophosphate is generated through base incorporation by a polymerase which further reacts with ATP sulfurylase to generate ATP in the presence of adenosine 5' phosphosulfate wherein the ATP generated may be consumed in a luciferase mediated reaction to generate a chemiluminescent signal. In another example, changes in an electrical current can be detected as a nucleic acid passes through a nanopore without the need for an illumination source.

In various embodiments, a data acquisition analysis and control unit 208 can monitor various system parameters. The system parameters can include temperature of various portions of instrument 200, such as sample processing unit 204 or reagent reservoirs, volumes of various reagents, the status of various system subcomponents, such as a manipulator, a stepper motor, a pump, or the like, or any combination thereof.

It will be appreciated by one skilled in the art that various embodiments of instrument 200 can be used to practice variety of sequencing methods including ligation-based methods, sequencing by synthesis, single molecule methods, nanopore sequencing, and other sequencing techniques.

In various embodiments, the sequencing instrument 200 can determine the sequence of a nucleic acid, such as a polynucleotide or an oligonucleotide. The nucleic acid can include DNA or RNA, and can be single stranded, such as ssDNA and RNA, or double stranded, such as dsDNA or a RNA/cDNA pair. In various embodiments, the nucleic acid can include or be derived from a fragment library, a mate pair library, a ChIP fragment, or the like. In particular embodiments, the sequencing instrument 200 can obtain the sequence information from a single nucleic acid molecule or from a group of substantially identical nucleic acid molecules.

In various embodiments, sequencing instrument 200 can output nucleic acid sequencing read data in a variety of different output data file types/formats, including, but not limited to: *.fasta, *.csfasta, *seq.txt, *qseq.txt, *.fastq, *.sff, *prb.txt, *.sms, *srs and/or *.qv.

### SYSTEM AND METHODS FOR IDENTIFYING SEQUENCE VARIATION

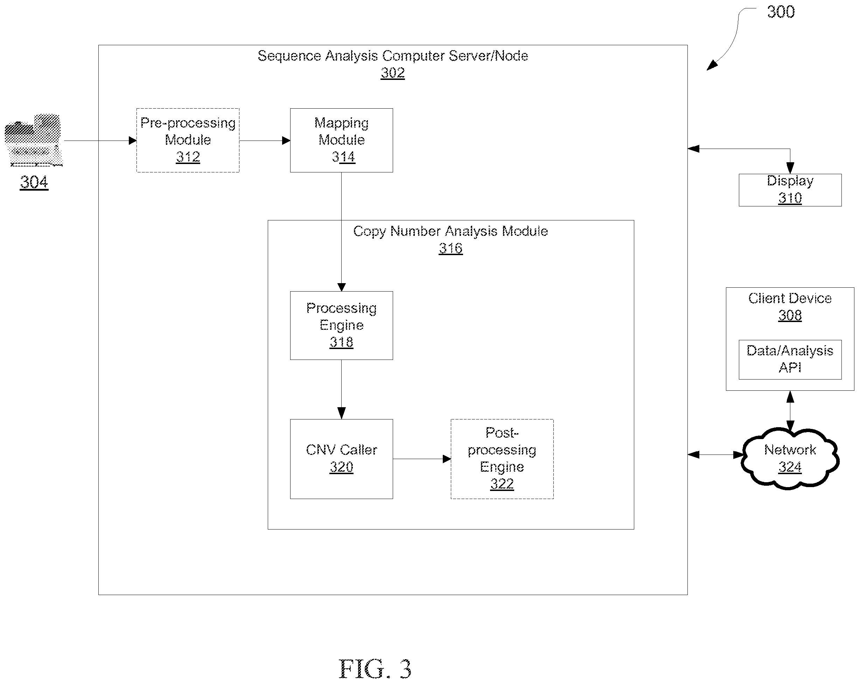

Figure 3 is a schematic diagram of a system for identifying variants, in accordance with various embodiments.

As depicted herein, variant analysis system 300 can include a nucleic acid sequence analysis device 304 (e.g., nucleic acid sequencer, real-time/digital/quantitative PCR instrument, microarray scanner, etc.), an analytics computing server/node/device 302, and a display 310 and/or a client device terminal 308.

In various embodiments, the analytics computing server/node/device 302 can be communicatively connected to the nucleic acid sequence analysis device 304, and client device terminal 308 via a network connection 324 that can be either a "hardwired" physical network connection (e.g., Internet, LAN, WAN, VPN, etc.) or a wireless network connection (e.g., Wi-Fi, WLAN, etc.).

In various embodiments, the analytics computing device/server/node 302 can be a workstation, mainframe computer, distributed computing node (such as, part of a "cloud computing" or distributed networking system), personal computer, mobile device, etc. In various embodiments, the nucleic acid sequence analysis device 304 can be a nucleic acid sequencer, real-time/digital/quantitative PCR instrument, microarray scanner, etc. It should be understood, however, that the nucleic acid sequence analysis device 304 can essentially be any type of instrument that can generate nucleic acid sequence data from samples obtained from an individual.

The analytics computing server/node/device 302 can be configured to host an optional pre-processing module 312, a mapping module 314, and a copy number analysis module 316.

Pre-processing module 312 can be configured to receive from the nucleic acid sequence analysis device 304 and perform processing steps, such as conversion from color space to base space or from flow space to base space, determining call quality values, preparing the read data for use by the mapping module 314, and the like.

The mapping module 314 can be configured to align (i.e., map) a nucleic acid sequence read to a reference sequence. Generally, the length of the sequence read is substantially less than the length of the reference sequence. In reference sequence mapping/alignment, sequence reads are assembled against an existing backbone sequence (e.g., reference sequence, etc.) to build a sequence that is similar but not necessarily identical to the backbone sequence. Once a backbone sequence is found for an organism, comparative sequencing or re-sequencing can be used to characterize the genetic diversity within the organism's species or between closely related species. In various embodiments, the reference sequence can be a whole/partial genome, whole/partial exome, etc.

In various embodiments, the sequence read and reference sequence can be represented as a sequence of nucleotide base symbols in base space. In various embodiments, the sequence read and reference sequence can be represented as one or more colors in color space. In various embodiments, the sequence read and reference sequence can be represented as nucleotide base symbols with signal or numerical quantitation components in flow space.

In various embodiments, the alignment of the sequence fragment and reference sequence can include a limited number of mismatches between the bases that comprise the sequence fragment and the bases that comprise the reference sequence. Generally, the sequence fragment can be aligned to a portion of the reference sequence in order to minimize the number of mismatches between the sequence fragment and the reference sequence.

The copy number analysis module 316 includes a processing engine 318, a copy number variant caller 320, and an optional post processing engine 322. In various embodiments, copy number analysis module 316 can be in communications with the mapping module 314. That is, copy number analysis module 316 can request and receive data and information (through, e.g., data streams, data files, text files, etc.) from mapping module 314.

The processing engine 318 can be configured to receive mapped reads from the mapping module 314, determine coverages for non-overlapping target regions of the genome (tiles), and normalize the tile coverages based on average or mode of the coverage across the tiles and the GC content. In various embodiments, the processing engine 318 can determine the normalized coverages for both a sample and a control.

CNV caller 320 can be configured to receive the normalized coverages from the processing engine 318, determine scores, such as likelihoods, for a tile to be present in various ploidy states, determine a maximum score path through the ploidy states across the tiles, and calculate score ratios, such as log likelihood ratios, of the maximum score path to the expected ploidy state and the closest scoring neighboring ploidy state. Additionally, the CNV caller 320 can identify copy number variants based on the maximum score ploidy states that can overcome a preset ploidy transition penalty. The transition penalty can be adjusted to accomplish desired sensitivity or specificity of the algorithm.

Post processing engine 322 can be configured to receive the copy number variants and the log likelihood ratios determined by the CNV caller 320 and perform additional processing steps, such as filtering copy number variants, and formatting the read data for display on display 310 or use by client device 308.

Client device 308 can be a thin client or thick client computing device. In various embodiments, client terminal 308 can have a web browser (e.g., INTERNET EXPLORER^{™}, FIREFOX^{™}, SAFARI^{™}, etc) that can be used to communicate information to and/or control the operation of the pre-processing module 312, mapping module 314, realignment engine 318, variant calling engine 320, and post processing engine 322 using a browser to control their function. For example, the client terminal 308 can be used to configure the operating parameters (e.g., match scoring parameters, annotations parameters, filtering parameters, data security and retention parameters, etc.) of the various modules, depending on the requirements of the particular application. Similarly, client terminal 308 can also be configure to display the results of the analysis performed by the variant calling module 316 and the nucleic acid sequencer 304.

It should be understood that the various data stores disclosed as part of system 300 can represent hardware-based storage devices (e.g., hard drive, flash memory, RAM, ROM, network attached storage, etc.) or instantiations of a database stored on a standalone or networked computing device(s).

It should also be appreciated that the various data stores and modules/engines shown as being part of the system 300 can be combined or collapsed into a single module/engine/data store, depending on the requirements of the particular application or system architecture. Moreover, in various embodiments, the system 300 can comprise additional modules, engines, components or data stores as needed by the particular application or system architecture.

In various embodiments, the system 300 can be configured to process the nucleic acid reads in color space. In various embodiments, system 300 can be configured to process the nucleic acid reads in base space. In various embodiments, system 300 can be configured to process the nucleic acid sequence reads in flow space. It should be understood, however, that the system 300 disclosed herein can process or analyze nucleic acid sequence data in any schema or format as long as the schema or format can convey the base identity and position of the nucleic acid sequence.

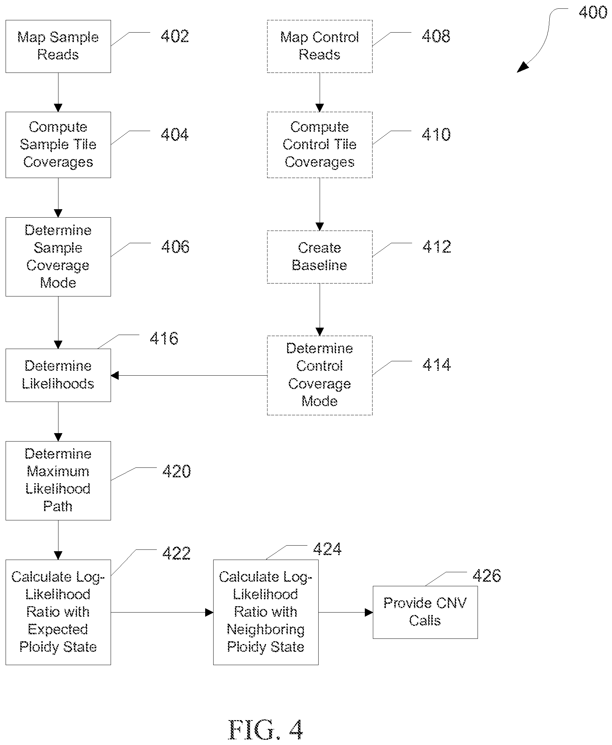

Figure 4 is an exemplary flow diagram showing a method 400 for identifying copy number variants, in accordance with various embodiments.

At 402, reads from the sample are mapped. At 404, the target region is divided into tiles, and the coverage of those tiles is determined. In various embodiments, the coverage is determined based on the number of reads that map to the tile and the number of bases in their overlaps. At 406, the mode of the tile coverage distribution can be determined based on the tile coverages across part of the sequence that has presumed single ploidy state. The mode will be the representative coverage for that ploidy state. In various embodiments such representative coverage can be determined also as a mean or a specific quantile (such as median) of the tile coverages.

In various embodiments, the coverage mode can be determined based on GC bias corrected coverages. For example, the mean coverages for a plurality of GC content bins (GC content of the tiles) can be calculated. In various embodiments, the GC bias distribution can be smoothed, such as using a LOESS (local regression) or LOWESS (locally weighted scatter plot smoothing) algorithm. A coverage scaling factor can be determined for a GC bin by dividing the overall mean coverage by the mean coverage of the GC bin. The tile coverage for a tile can be multiplied by the GC bias correction factor from the bin with corresponding GC content to obtain a GC bias corrected coverage for the tile. The GC bias corrected coverage can be used in the computation of the coverage modes.

In various embodiments, tiles, coverage, and modes can be determined on a per chromosome basis. For example, the target regions of chromosome 1 can be divided into a first set of tiles and chromosome 2 can be divided into a second set of tiles. The coverages and coverage mode of the first set of tiles can be determined separately from the coverages and coverage mode of the second set of tiles.

In various embodiments, target regions can be amplified in a plurality of multiplex PCR reactions. For example, a first set of target regions can be amplified in a first PCR reaction vessel, and a second set of target regions can be amplified in a second PCR reaction vessel. Coverages and coverage modes can be determined on a per PCR reaction vessel, such that the first set of target regions can be assigned to a first set of tiles and the second set of target regions can be assigned to a second set of tiles. The coverages and coverage mode of the first set of tiles can be determined separately from the coverages and coverage mode of the second set of tiles. These sets of tiles can overlap if some tiles span target regions belonging to several PCR pools. The algorithm can assign each read to a specific PCR pool, such as, for instance, by finding a target region with maximum overlap with the read.

At 408, control reads are mapped to the reference genome. At 410, the target region is divided into tiles, and the coverage of those tiles by the control is determined. In various embodiments, the coverage is determined based on the number of control reads that map to the tile and the number of bases that overlap with the tile. At 412, a baseline is created, and at 414, the mode of the baseline coverage is determined based on the coverage across the baseline. The baseline can be created from a single control sample, however, in some embodiments the baseline can be created by adding coverages from plurality of control samples and by adjusting the coverages by their known ploidy information. In various embodiments, the control samples' target regions can be amplified in a plurality of multiplex PCR reactions and the composite baseline coverages and their coverage modes can be determined per PCR pool.

At 414, the coverages are normalized so that the coverages are equivalent between the sample and baseline. In various embodiments, the equivalence can be achieved by scaling the coverages of both the sample and the baseline such that the coverage mode becomes 2. As the mode corresponds to a representative coverage for the diploid state, the normalized coverages approximate the ploidy value. Having a common coverage scale for the sample and the baseline allows a direct comparison of the sample and baseline coverage when calculating a score, such as a likelihood of the sample being in any particular ploidy state.

At 416, a score for a tile being in various ploidy states is calculated. The score can be a function or ad-hoc rule that can discriminate between the true and other ploidy states. In various embodiments, the score can be calculated for a range of ploidy states, such as a range from a ploidy of 1 through 10. In an exemplary embodiment, the score for a ploidy state can be calculated as a likelihood using the equation L = N( S-C, 0, Sd ), where S is the normalized sample coverage for the tile, C is the scaled baseline coverage for the tile in the explored ploidy state, and Sd is the standard deviation of the coverage. The standard deviation can be made dependent on the sample and control coverage and predetermined using sequencing of technical sample replicates. In various embodiments, the scaled baseline coverage can be determined by scaling the normalized baseline to an explored ploidy state. For example, the normalized baseline coverage for a diploid region can be approximately 2, and when exploring a ploidy state of 3 (triploid), the normalized baseline coverage can be multiplied by 3/2, such that the scaled baseline coverage is approximately 3. Thus, for a tile that is in a trip loid region in the sample, the difference between the normalized sample coverage and the scaled baseline coverage could be greater when scoring a ploidy state of 2 or 4 than when scoring a ploidy state of three.

In various embodiments, such as when determining copy number variation for a sample from a cancerous tumor, multiple subpopulations of cells may exist in the sample. For example, a sample from a cancer biopsy may include normal cells as well as cancerous cells and have an effective ploidy state that represents a weighted average of the ploidy state of the normal cells and the cancerous cells. To identify copy number changes for specific genes in such a sample, scores can be calculated for none-integer ploidy states. For example, scores can be calculated over a range of values in steps of one tenth, such as 2.0, 2.1, 2.2, 2.3, etc.

At 420, a maximum score path through a ploidy state for each tile is determined. In various embodiments, scoring of the path can include a summation of the scores for each ploidy state along the path and a transition penalty for each pair of neighboring tiles where the ploidy state changes. The maximum score path can then be determined using dynamic programming algorithm, such as the Viterbi algorithm in the implementations of Hidden Markov Models. In an exemplary embodiment, the maximum score path can be a maximum likelihood path calculated by summing the log-likelihoods of the ploidy states along the path and the transition penalty.

The transition penalty can be a deterrent to changing copy-number state for small segments, unless there is an overriding support from the state likelihoods of the tiles in the segment which will outweigh the transition penalty. In various embodiments, the transition penalty can be a function of the log of the probability that the copy-number state changes for any given random tile. Making the transition probability smaller will result in calling only larger CNV segments, or only segments with greater support for the changed state (greater difference in copy number). Therefore the transition penalty can be adjusted to achieve a desired sensitivity or specificity.

The transition probability can increase for larger gaps between tiles. These gaps can arise from un-amplified (un-sequenced) part of the genome that can be skipped in the algorithm. After a sufficiently large gap, the copy-number state of the tile before the gap may have no information about the copy-number state of a tile after the gap. Therefore the transition probability can become equal for all copy-number states after the gap. The increase to this all-equal probability can be exponential and therefore can be estimated by linear increase in the log space.

At 422, a score ratio, such as a log-likelihood ratio, of the maximum likelihood path and the expected ploidy state can be calculated. For example, regions on chromosome 1 can be expected to have a ploidy of 2 and regions on the X and Y chromosomes can have a ploidy of 1 and 1 for males, and 2 and 0, respectively, for females. In various embodiments, the expected ploidy state for the target regions can be provided. This score ratio can serve as a confidence metric for the existence of a CNV segment with ploidy that is different from the expected ploidy state.

At 424, a score ratio, such as a log-likelihood ratio, of the maximum likelihood path and the most likely neighboring state can be calculated. The score of the neighboring states, +1 and -1 ploidy, can be compared to identify the most likely neighboring state for the CNV segment, and a score ratio can be determined for the most likely neighboring state path. Such ratio will determine the degree of accuracy of the assigned ploidy number.

In various embodiments, a median absolute pairwise difference (MAPD) can be calculated for the ratio of the normalized sample coverage and the baseline for each pair of adjacent tiles. The differences can be averaged over a region of the genome, such as for a gene. Alternatively the differences can be averaged over the genome, the sequenced portion of the genome, or a region of uniform ploidy state. A low MAPD value can be indicative of copy number data for consecutive tiles being tightly clustered around a single value. A high MAPD value can indicate significant variability in the copy number data for consecutive tiles, and thus a lower confidence in the copy number call.

At 426, the ploidy states and the score ratios can be provided. The score ratios can be used as a measure of confidence in the copy number call for a tile.

In various embodiments, a control is sequenced substantially simultaneously with the sample, such as sequencing the control and the sample in the same run. In exemplary embodiments, control reads and sample reads can be identified based on a barcode or other known sequence attached to the fragments prior to sequencing to distinguish the control from the sample.

In various embodiments, large scale, low pass sequencing can be performed on a sample. For example, the sample can be a whole genome, an exome, or the like. The large scale, low pass sequencing can generate reads at a relatively low coverage. For example, the average coverage can be at least about 0.00001×, such as greater than about 0.000lx, even greater than about 0.001x. In particular embodiments, the coverage may be at a level insufficient to ensure complete coverage of the genome, such as less than about 10×, such as less than about 1×, even less than about 0.1×. The determination of the copy number can be performed with a large tile size, such as about 2 megabases. Generally, the tile size should be sufficient to have an average of at least about 100 reads per tile. Additionally, the likelihoods can be determined based on an informatics baseline. For example, the informatics baseline can be precomputed by combining data from multiple sequencing runs, either from the same sample or multiple samples with a known copy number profile. Using large tile sizes and low pass sequencing can detect the gain or loss of a chromosome and can provide information on large scale chromosomal rearrangements.

In various embodiments, selective sequencing can be performed on a sample. For example, selected regions of a genome can be amplified using PCR, such as multiple PCR or the genome can be fragmented and fragments from selected regions can be isolated, such as by binding to a probe array. A matched control can be prepared in the same manner as the sample, and the sample and matched control can be sequenced separately, or during the same sequencing run. Selective sequencing can result in a relatively high coverage of the selected portions. Therefore, smaller tile sizes can be used, such as about 100 bases. The likelihoods can be determined from on a baseline calculated from the matched control. Higher coverage sequencing with smaller tile sizes can detect smaller regions of high or low copy number, such as gene duplications.

In various embodiments, rather than dividing the genome into discrete equal sized tiles, the analysis can be performed on each amplified region. The coverage for an amplified region can be determined based on the number of reads from an amplicon of the region. While the amplified regions may partially overlap, overlapping regions will generally be amplified in separate PCR pools and can be distinguished based on the sequence of non-overlapping portions of the reads.

In various embodiments, the methods of the present teachings may be implemented in a software program and applications written in conventional programming languages such as C, C++, etc.

Further, in describing various embodiments, the specification may have presented a method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims.

The embodiments described herein, can be practiced with other computer system configurations including hand-held devices, microprocessor systems, microprocessor-based or programmable consumer electronics, minicomputers, mainframe computers and the like. The embodiments can also be practiced in distributing computing environments where tasks are performed by remote processing devices that are linked through a network.

It should also be understood that the embodiments described herein can employ various computer-implemented operations involving data stored in computer systems. These operations are those requiring physical manipulation of physical quantities. Usually, though not necessarily, these quantities take the form of electrical or magnetic signals capable of being stored, transferred, combined, compared, and otherwise manipulated. Further, the manipulations performed are often referred to in terms, such as producing, identifying, determining, or comparing.

Any of the operations that form part of the embodiments described herein are useful machine operations. The embodiments, described herein, also relate to a device or an apparatus for performing these operations. The systems and methods described herein can be specially constructed for the required purposes or it may be a general purpose computer selectively activated or configured by a computer program stored in the computer. In particular, various general purpose machines may be used with computer programs written in accordance with the teachings herein, or it may be more convenient to construct a more specialized apparatus to perform the required operations.

Certain embodiments can also be embodied as computer readable code on a computer readable medium. The computer readable medium is any data storage device that can store data, which can thereafter be read by a computer system. Examples of the computer readable medium include hard drives, network attached storage (NAS), read-only memory, random-access memory, CD-ROMs, CD-Rs, CD-RWs, magnetic tapes, and other optical and non-optical data storage devices. The computer readable medium can also be distributed over a network coupled computer systems so that the computer readable code is stored and executed in a distributed fashion.

## Claims

1. A method of providing copy number determinations, comprising:
performing a multiplex amplification on a sample to generate a set of sample amplicons;
performing a multiplex amplification on a matched control to generate a set of control amplicons;
joining adaptors having a first barcode sequence to the sample amplicons to create a sample library;
joining adaptors having a second barcode sequence to the control amplicons to create a control library;
sequencing the sample and control libraries substantially simultaneously to avoid intra-run sequencing variations to generate a plurality of reads;
identifying reads as either sample reads or control reads based on the presence of the first or second barcode sequence;
mapping the sample reads and control reads to a reference genome;
computing a sample coverage for a plurality of tiles based on the sample reads that map to the tiles, wherein the tiles are non-overlapping target regions of the reference genome and each tile comprises at least 100 bases and a coverage for a tile is determined based on a number of reads that map to the tile and on a number of bases in overlaps of the reads;
computing a baseline coverage for the tiles based on the control reads that map to the tiles;
normalizing the sample coverage for a tile based on the sample coverage mode and normalizing the baseline coverage for the tile based on the control coverage mode across the plurality of tiles;
determining a score function for each tile of the plurality of tiles being in a plurality of ploidy states based on the normalized sample coverage and the normalized baseline coverage for the tiles, wherein the score function is a likelihood function;
determining a maximum likelihood path across the tiles and through the ploidy states; and
providing a copy number determination based on the maximum likelihood path.

2. The method of claim 1, wherein the sample coverage mode and the control coverage mode is corrected for GC bias.

3. The method of claim 1, wherein the score function for a tile being in a ploidy state is based on the difference between the normalized coverage and a scaled baseline coverage adjusted to the explored ploidy state.

4. The method of claim 1, wherein the likelihood is determined using the equation L = N( S-C, 0, Sd ), where S is the normalized sample coverage for the tile, C is a scaled baseline coverage for the tile adjusted to the explored ploidy state, and Sd is the standard deviation of the coverage difference.

5. The method of claim 1, further comprising determining a score ratio of the value of the maximum score path for a tile of the plurality of tiles to the expected ploidy state of the tile of the plurality of tiles.

6. The method of claim 1, further comprising determining a score ratio of the value of the maximum score path for a tile of the plurality of tiles to the most likely neighboring state of the tile of the plurality of tiles.

7. A system for providing copy number determinations, comprising:
a nucleic acid sequencing analysis device operable to sequence sample and control libraries substantially simultaneously to avoid intra-run sequencing variations to generate a plurality of reads, wherein the reads are identified as either sample reads or control reads based on the presence of a first or a second barcode sequence, wherein the sample libraries have been created by joining adaptors having the first barcode sequence to sample amplicons that have been generated by performing a multiplex amplification on a sample, and wherein the control libraries have been created by joining adaptors having the second barcode sequence to control amplicons that have been generated by performing a multiplex amplification on a matched control;
a mapping engine operable to map the sample reads and control reads to a reference genome to determine a genomic start position and an flow end position; and
a copy number analysis module comprising:
a processing engine operable to:
determine a sample coverage for tiles based on the sample reads that map to the tiles, wherein the tiles are non-overlapping target regions of the reference genome and each tile comprises at least 100 bases and a coverage for a tile is determined based on a number of reads that map to the tile and on a number of bases in overlaps of the reads;
determine a baseline coverage for the tiles based on the control reads that map to the tiles; and
normalize the sample coverage for a tile based on the sample coverage mode and normalize the baseline coverage for the tile based on the control coverage mode across the plurality of tiles; and
a copy number variant caller operable to determine a score function for each tile of the tiles to be present in a plurality of ploidy states based on the normalized sample coverage and the normalized baseline coverage for the tiles, determine a maximum likelihood path across the tiles through the ploidy states, and provide a copy number determination based on the maximum likelihood path.

8. The system of claim 7, wherein the score function is a likelihood function and wherein the likelihood for a tile being in a ploidy state is based on the difference between the normalized coverage and a scaled baseline coverage scaled to the ploidy state.

9. The system of claim 8, wherein the likelihood is determined using the equation L = N( S-C, 0, Sd ), where S is the normalized sample coverage for the tile, C is the scaled baseline coverage for the tile, and Sd is the standard deviation of the coverage difference.

10. The system of claim 7, wherein the copy number analysis module further includes a post-processing module operable to determine a score ratio of the value of the maximum score path for a tile of the plurality of tiles to the expected ploidy state of the tile of the plurality of tiles.

11. The system of claim 7, wherein the copy number analysis module further includes a post-processing module operable to determine a score ratio of the value of the maximum score path for a tile of the plurality of tiles to the most likely neighboring ploidy state of the tile of the plurality of tiles.

## Patentansprüche

1. Verfahren zum Bereitstellen von Kopienzahlbestimmungen, umfassend:
Durchführen einer Multiplex-Amplifikation auf einer Probe, um einen Satz von Probenamplikons zu erzeugen;
Durchführen einer Multiplex-Amplifikation auf einer angepassten Kontrolle, um einen Satz von Kontrollamplikons zu erzeugen;
Verbinden von Adaptern, die eine erste Barcode-Sequenz mit den Probenamplikons aufweisen, um eine Probenbibliothek zu erstellen;
Verbinden von Adaptern, die eine zweite Barcode-Sequenz mit den Kontrollamplikons aufweisen, um eine Kontrollbibliothek zu erstellen;
Sequenzieren der Proben- und Kontrollbibliotheken im Wesentlichen gleichzeitig, um innerhalb verlaufende Sequenzierungsvariationen zu vermeiden, um eine Vielzahl von Lesungen zu erzeugen;
Identifizieren von Lesungen als entweder Probenlesungen oder Kontrolllesungen basierend auf dem Vorhandensein der ersten oder der zweiten Barcode-Sequenz;
Zuordnen der Probenlesungen und der Kontrolllesungen zu einem Referenzgenom;
Berechnen einer Probenabdeckung für eine Vielzahl von Kacheln basierend auf den Probenlesungen, die zu den Kacheln zuordnen, wobei die Kacheln nicht überlappende Zielbereiche des Referenzgenoms sind und jede Kachel mindestens 100 Basen umfasst und eine Abdeckung für eine Kachel basierend auf einer Anzahl von Lesungen, die der Kachel zugeordnet sind, und auf einer Anzahl von Basen in Überlappungen der Lesungen bestimmt wird;
Berechnen einer Basislinienabdeckung für die Kacheln basierend auf den Kontrolllesungen, die den Kacheln zugeordnet sind;
Normalisieren der Probenabdeckung für eine Kachel basierend auf dem Probenabdeckungsmodus und Normalisieren der Basislinienabdeckung für die Kachel basierend auf dem Kontrollabdeckungsmodus über die Vielzahl von Kacheln hinweg;
Bestimmen einer Bewertungsfunktion für jede Kachel der Vielzahl von Kacheln, die sich in einer Vielzahl von Ploidiezuständen befindet, basierend auf der normalisierten Probenabdeckung und der normalisierten Basislinienabdeckung für die Kacheln, wobei die Bewertungsfunktion eine Wahrscheinlichkeitsfunktion ist;
Bestimmen eines maximalen Wahrscheinlichkeitswegs über die Kacheln hinweg und über die Ploidiezustände; und
Bereitstellen einer Kopienzahlbestimmung basierend auf dem maximalen Wahrscheinlichkeitsweg.

2. Verfahren nach Anspruch 1, wobei der Probenabdeckungsmodus und der Kontrollabdeckungsmodus für die GC-Verzerrung korrigiert werden.

3. Verfahren nach Anspruch 1, wobei die Bewertungsfunktion für eine Kachel, die sich in einem Ploidiezustand befindet, auf der Differenz zwischen der normalisierten Abdeckung und einer skalierten Basislinienabdeckung basiert, die an den untersuchten Ploidiezustand angepasst ist.

4. Verfahren nach Anspruch 1, wobei die Wahrscheinlichkeit unter Verwendung der Gleichung L=N(S-C, 0, Sd) bestimmt wird, wobei S die normalisierte Probenabdeckung für die Kachel ist, C eine skalierte Basislinienabdeckung für die Kachel ist, die an den untersuchten Ploidiezustand angepasst ist, und Sd die Standardabweichung der Abdeckungsdifferenz ist.

5. Verfahren nach Anspruch 1, ferner umfassend das Bestimmen eines Bewertungsverhältnisses des Werts des maximalen Bewertungswegs für eine Kachel der Vielzahl von Kacheln zum erwarteten Ploidiezustand der Kachel der Vielzahl von Kacheln.

6. Verfahren nach Anspruch 1, ferner umfassend das Bestimmen eines Bewertungsverhältnisses des Werts des maximalen Bewertungswegs für eine Kachel der Vielzahl von Kacheln zum wahrscheinlichsten Nachbarzustand der Kachel der Vielzahl von Kacheln.

7. System zum Bereitstellen von Kopienzahlbestimmungen, umfassend:
eine Nukleinsäuresequenzierungsanalysevorrichtung, die betriebsfähig ist, um Proben- und Kontrollbibliotheken im Wesentlichen gleichzeitig zu sequenzieren, um die innerhalb verlaufenden Sequenzierungsvariationen zu vermeiden, um eine Vielzahl von Lesungen zu erzeugen, wobei die Lesungen entweder als Probenlesungen oder Kontrolllesungen basierend auf dem Vorhandensein einer ersten oder einer zweiten Barcode-Sequenz identifiziert werden, wobei die Probenbibliotheken durch Verbinden von Adaptern, die die erste Barcode-Sequenz aufweisen, mit Probenamplikons erstellt wurden, die durch Durchführen einer Multiplex-Amplifikation auf einer Probe erzeugt wurden, und wobei die Kontrollbibliotheken durch Verbinden von Adaptoren, die die zweite Barcode-Sequenz aufweisen, mit Probenamplikons erstellt wurden, die durch Durchführen einer Multiplex-Amplifikation auf einer übereinstimmenden Kontrolle erzeugt wurden;
eine Zuordnungsmaschine, die betriebsfähig ist, um die Probenlesungen und Kontrolllesungen zu einem Referenzgenom zuzuordnen, um eine genomische Startposition und eine Flussendposition zu bestimmen; und
ein Kopienzahlanalysemodul, umfassend:
eine Verarbeitungsmaschine, die betriebsfähig ist zum:
Bestimmen einer Probenabdeckung für Kacheln basierend auf den Probenlesungen, die zu den Kacheln zuordnen, wobei die Kacheln nicht überlappende Zielbereiche des Referenzgenoms sind und jede Kachel mindestens 100 Basen umfasst und eine Abdeckung für eine Kachel basierend auf einer Anzahl von Lesungen, die zu der Kachel zuordnen, und auf einer Anzahl von Basen in Überlappungen der Lesungen bestimmt wird;
Bestimmen einer Basislinienabdeckung für die Kacheln basierend auf den Kontrolllesungen, die zu den Kacheln zuordnen; und
Normalisieren der Probenabdeckung für eine Kachel basierend auf dem Probenabdeckungsmodus und Normalisieren der Basislinienabdeckung für die Kachel basierend auf dem Kontrollabdeckungsmodus über die Vielzahl von Kacheln hinweg; und
einen Kopienzahlvariantenaufrufer, der betriebsfähig ist, um eine Bewertungsfunktion für jede Kachel der Kacheln zu bestimmen, um in einer Vielzahl von Ploidiezuständen basierend auf der normalisierten Probenabdeckung und der normalisierten Basislinienabdeckung für die Kacheln vorhanden zu sein, einen maximalen Wahrscheinlichkeitsweg über die Kacheln hinweg über die Ploidiezustände zu bestimmen und eine Kopienzahlbestimmung basierend auf dem maximalen Wahrscheinlichkeitsweg bereitzustellen.

8. System nach Anspruch 7, wobei die Bewertungsfunktion eine Wahrscheinlichkeitsfunktion ist und wobei die Wahrscheinlichkeit für eine Kachel, die sich in einem Ploidiezustand befindet, auf der Differenz zwischen der normalisierten Abdeckung und einer skalierten Basislinienabdeckung basiert, die auf den Ploidiezustand skaliert wird.

9. System nach Anspruch 8, wobei die Wahrscheinlichkeit unter Verwendung der Gleichung L=N(S-C, 0, Sd) bestimmt wird, wobei S die normalisierte Probenabdeckung für die Kachel ist, C die skalierte Basislinienabdeckung für die Kachel ist und Sd die Standardabweichung der Abdeckungsdifferenz ist.

10. System nach Anspruch 7, wobei das Kopienzahlanalysemodul ferner ein Nachverarbeitungsmodul einschließt, das betriebsfähig ist, um ein Bewertungsverhältnis des Werts des maximalen Bewertungswegs für eine Kachel der Vielzahl von Kacheln zum erwarteten Ploidiezustand der Kachel der Vielzahl von Kacheln zu bestimmen.

11. System nach Anspruch 7, wobei das Kopienzahlanalysemodul ferner ein Nachverarbeitungsmodul einschließt, das betriebsfähig ist, um ein Bewertungsverhältnis des Werts des maximalen Bewertungswegs für eine Kachel der Vielzahl von Kacheln zum wahrscheinlichsten benachbarten Ploidiezustand der Kachel der Vielzahl von Kacheln zu bestimmen.

## Revendications

1. Procédé de fourniture de déterminations de nombre de copies, comprenant :
la réalisation d'une amplification multiplex sur un échantillon pour générer un ensemble d'amplicons d'échantillon ;
la réalisation d'une amplification multiplex sur un contrôle apparié pour générer un ensemble d'amplicons de contrôle ;
l'assemblage d'adaptateurs ayant une première séquence de codes-barres aux amplicons d'échantillon pour créer une bibliothèque d'échantillons ;
l'assemblage d'adaptateurs ayant une seconde séquence de codes-barres aux amplicons de contrôle pour créer une bibliothèque de contrôle ;
le séquençage des bibliothèques d'échantillon et de contrôle sensiblement simultanément pour éviter des variations de séquençage intra-course pour générer une pluralité de lectures ;
l'identification de lectures comme des lectures d'échantillon ou des lectures de contrôle sur la base de la présence de la première ou de la seconde séquence de codes-barres ;
le mappage des lectures d'échantillon et des lectures de contrôle à un génome de référence ;
le calcul d'une couverture d'échantillon pour une pluralité de mosaïques sur la base des lectures d'échantillon qui correspondent aux mosaïques, dans lequel les mosaïques sont des régions cibles du génome de référence qui ne se chevauchent pas et chaque mosaïque comprend au moins 100 bases et une couverture pour une mosaïque est déterminée sur la base d'un nombre de lectures qui correspondent à la mosaïque et d'un nombre de bases dans les chevauchements des lectures ;
le calcul d'une couverture de ligne de base pour les mosaïques sur la base des lectures de contrôle qui correspondent aux mosaïques ;
la normalisation de la couverture d'échantillon pour une mosaïque sur la base du mode de couverture d'échantillon et la normalisation de la couverture de ligne de base pour la mosaïque sur la base du mode de couverture de contrôle à travers la pluralité de mosaïques ;
la détermination d'une fonction de score pour chaque mosaïque de la pluralité de mosaïques dans une pluralité d'états de ploïdie sur la base de la couverture d'échantillon normalisée et de la couverture de référence normalisée pour les mosaïques, dans lequel la fonction de score est une fonction de probabilité ;
la détermination d'un chemin de probabilité maximale à travers les mosaïques et par les états de ploïde ; et
la fourniture d'une détermination de nombre de copies sur la base du chemin de probabilité maximale.

2. Procédé selon la revendication 1, dans lequel le mode de couverture d'échantillon et le mode de couverture de contrôle sont corrigés pour un biais CG.

3. Procédé selon la revendication 1, dans lequel la fonction de score pour une mosaïque étant dans un état de ploïdie est basée sur la différence entre la couverture normalisée et une couverture de ligne de base mise à l'échelle ajustée à l'état de ploïdie explorée.

4. Procédé selon la revendication 1, dans lequel la probabilité est déterminée à l'aide de l'équation L = N( S-C, 0, Sd ), où S est la couverture d'échantillon normalisée pour la mosaïque, C est une couverture de ligne de base mise à l'échelle pour la mosaïque ajustée à l'état de ploïdie explorée, et Sd est l'écart type de la différence de couverture.

5. Procédé selon la revendication 1, comprenant en outre la détermination d'un rapport de score de la valeur du chemin de score maximal pour une mosaïque de la pluralité de mosaïques à l'état de ploïdie attendu de la mosaïque de la pluralité de mosaïques.

6. Procédé selon la revendication 1, comprenant en outre la détermination d'un rapport de score de la valeur du chemin de score maximal pour une mosaïque de la pluralité de mosaïques à l'état voisin le plus probable de la mosaïque de la pluralité de mosaïques.

7. Système permettant de fournir des déterminations de nombre de copies, comprenant :
un dispositif d'analyse de séquençage d'acide nucléique pouvant être utilisé pour séquencer des bibliothèques d'échantillon et de contrôle sensiblement simultanément pour éviter des variations de séquençage intra-course pour générer une pluralité de lectures, dans lequel les lectures sont identifiées comme étant soit des lectures d'échantillon, soit des lectures de contrôle basées sur la présence d'une première ou d'une seconde séquence de codes-barres, dans lequel les bibliothèques d'échantillons ont été créées par assemblage des adaptateurs ayant la première séquence de codes-barres à des amplicons d'échantillons qui ont été générés par réalisation d'une amplification multiplex sur un échantillon, et dans lequel les bibliothèques de contrôle ont été créées par réalisation d'une amplification multiplex sur un échantillon, et dans lequel les bibliothèques de contrôle ont été créées par réalisation d'une amplification multiplex sur un contrôle apparié ;
un moteur de mappage pouvant être utilisé pour mapper les lectures d'échantillon et les lectures de contrôle à un génome de référence pour déterminer une position de départ génomique et une position de fin de flux ; et
un module d'analyse du nombre de copies comprenant :
un moteur de traitement pouvant être utilisé pour :
déterminer une couverture d'échantillon pour des mosaïques sur la base des lectures d'échantillon qui correspondent aux mosaïques, dans lequel les mosaïques sont des régions cibles du génome de référence ne se chevauchent pas et chaque mosaïque comprend au moins 100 bases et une couverture pour une mosaïque est déterminée sur la base d'un nombre de lectures qui correspondent à la mosaïque et d'un nombre de bases dans les chevauchements des lectures ;
déterminer une couverture de ligne de base pour les mosaïques sur la base des lectures de contrôle qui correspondent aux mosaïques ; et
normaliser la couverture d'échantillon pour une mosaïque sur la base du mode de couverture d'échantillon et normaliser la couverture de ligne de base pour la mosaïque sur la base du mode de couverture de contrôle à travers la pluralité de mosaïques ; et
un programme d'appel de variante du nombre de copies pouvant être utilisé pour déterminer une fonction de score pour chaque mosaïque des mosaïques devant être présentes dans une pluralité d'états de ploïdie sur la base de la couverture d'échantillon normalisée et de la couverture de référence normalisée pour les mosaïques, déterminer un chemin de probabilité maximale à travers les mosaïques par l'intermédiaire des états de ploïdie, et fournir une détermination de nombre de copies sur la base du chemin de probabilité maximale.

8. Système selon la revendication 7, dans lequel la fonction de score est une fonction de probabilité et dans lequel la probabilité pour une mosaïque étant dans un état de ploïdie est basée sur la différence entre la couverture normalisée et une couverture de ligne de base mise à l'échelle, mise à l'échelle à l'état de ploïdie.

9. Système selon la revendication 8, dans lequel la probabilité est déterminée à l'aide de l'équation L = N( S-C, 0, Sd ), où S est la couverture d'échantillon normalisée pour la mosaïque, C est la couverture de ligne de base mise à l'échelle pour la mosaïque, et Sd est l'écart type de la différence de couverture.

10. Système selon la revendication 7, dans lequel le module d'analyse de nombre de copies comporte en outre un module de post-traitement pouvant être utilisé pour déterminer un rapport de score de la valeur du chemin de score maximal pour une mosaïque de la pluralité de mosaïques à l'état de ploïdie attendu de la mosaïque de la pluralité de mosaïques.

11. Système selon la revendication 7, dans lequel le module d'analyse de nombre de copies comporte en outre un module de post-traitement pouvant être utilisé pour déterminer un rapport de score de la valeur du chemin de score maximal pour une mosaïque de la pluralité de mosaïques à l'état de ploïdie voisin le plus probable de la mosaïque de la pluralité de mosaïques.
